# EUROPEAN PATENT SPECIFICATION

(11) **EP 0 491 094 B1**
(45) Date of publication and mention of the grant of the patent: **11.09.1996**
(21) Application number: 90313735.4
(22) Date of filing: 17.12.1990
(51) Int. Cl.: A61K 31/27

(54) **Felbamate for treating Lennox-Gastaut syndrome**
Felbamat zur Behandlung der Lennox-Gastaut syndrome
Felbamate pour le traitement du syndrom de Lennox-Gastaut

(43) Date of publication of application: 24.06.1992
(73) Proprietor: CARTER-WALLACE, INC., New York, N.Y. 10105 (US)
(72) Inventor: Sofia, Robert Duane, Willingboro, New Jersey08846 (US)
(74) Representative: W.P. Thompson & Co.

(56) References cited:
- EPILEPSIA, vol. 30, no. 5, 1989, page 661; W.H. THEODORE et al.: "Felbamate: A double-blind placebo-controlled three-period crossover trial"
- EPILEPSIA, vol. 30, no. 5, 1989, pages 661-662; I.E. LEPPIK et al.: "Felbamate in partial seizures: Results of phase II clinical trial"
- EPILEPSIA, vol. 27, no. 5, 1986, page 649; P.H. SHERIDAN et al.: "Open pilot study of felbamate (ADD 03055) in partial seizures"
- NEUROLOGY, vol. 39, suppl. 1, March 1989, page 120, abstract no. PP43; M.L. MILLER et al.: "Successful withdrawal of phenytoin in refractory patients with epilepsy"
- EPILEPSIA, vol. 27, no. 1, 1986, pages 27-34; E.A. SWINYARD et al.: "Comparative anticonvulsant activity and neurotoxicity of felbamate and four prototype antiepileptic drugs in mice and rats"

## Description

The present invention relates to pharmaceutical compositions containing 2-phenyl-1,3-propanediol dicarbamate as an active component and to methods for the prevention and control of epileptic seizures by the use of such compositions.

More particularly, the present invention further relates to methods for increasing epileptic seizure threshold and the prevention of epileptic seizure spread through the administration of therapeutic compositions which contain as an active ingredient 2-phenyl-1,3-propanediol dicarbamate commonly known as Felbamate.

Felbamate is a well known pharmaceutical compound having been described in US-A- 2 884 444 and US-A-4 868 327.

It has also been described to be effective in the treatment of epilepsy (Epilepsia, vol. 30, no. 5 1989, p. 661; Epilepsia, vol. 30, no. 5; 1989, p. 661-662; Epilepsia, vol. 27, no. 5, 1986, p. 649; Neurology, vol. 27, suppl. 1, 1989, p. 120, Abs. No. pp 43)

Epilepsy, a disease which has been characterized as a paroxysmal, self-sustaining and self-limited cerebral dysrhythmia, genetic or acquired in origin and physiologic or organic in mechanism is generally divided into four main types based on the type of seizure that occurs in those afflicted with the disease.

Based on clinical and electroencephalographic observations, the four general subdivisions of epilepsy are:
1. Grand mal
2. Petit mal
3. Psychomotor
4. Autonomic

Those afflicted with epilepsy may present with any one of or a mixture of the foregoing forms of the disease.

In theory, it is believed that anti-epileptic drugs act to prevent or control seizures by acting on the seizure focus which may be a collection of pathologically altered neurons or normal cells having restricted vascular supply or an injured area in which the neurons of a nerve net have been destroyed.

Up to the present time, all drugs used in the treatment of epilepsy function as prophylactics against the symptoms of epilepsy, i.e., the reduction and control of epileptic seizures as opposed to being curatives.

Although it is generally recognized that approximately 50% of epileptic patients can be controlled with presently available anti-epileptic medications, there is a continuing long felt need for more selective and less toxic anti-epileptic drugs. The desiratum of the art has been to provide a non-toxic, non-sedative, long-acting and highly effective anti-epileptic drug.

Phenytoin and carbamazepine are presently the drugs of choice for control of both generalized tonic-clonic (grand mal) and complex partial (temporal lobe) epileptic seizures.

In addition to gingival hyperplasia and hirsutism peculiar to phenytoin, both drugs have been reported to induce cerebellar-vestibular effects, skin disorders, hepatic deficiencies and congenital abnormalities. The foregoing toxicity profile for both phenytoin and carbamazepine clearly demonstrates a need for less toxic substances for use as anti-epileptic medications.

According to the present invention there is provided the use of 2-phenyl-1,3-propanediol dicarbamate for the manufacture of a medicament for the prevention and control of seizures associated with Lennox-Gastaut syndrome.

It has been found that felbamate chemically described as 2-phenyl-1,3-propanediol dicarbamate is a compound which has demonstrated superior properties when compared to prototype drugs, i.e. phenytoin with respect to increasing seizure threshold and preventing seizure spread.

The compositions for the treatment of epilepsy may take any of a variety of forms although they are intended primarily for oral use and are suitable for forming into pills, capsules and tablets by well-known practices. When the active ingredient is in the form of a solid, a typical tablet composition comprises 500 milligrams of 2-phenyl-1,3-propanediol dicarbamate intermixed in a dry pulverulent state with suitable solid carriers and diluents.

In general, an effective daily dose of the active ingredient is in the range of from 100 milligrams to 5 grams.

Solid carriers and diluents suitable for use include sugars such as, for example, lactose and sucrose; cellulose derivatives such as, for example, carboxymethyl cellulose, ethyl cellulose and methyl cellulose; gelatin including hard and soft gelatin capsules; talc; cornstarch; stearic acid; and magnesium stearate.

The percentage of 2-phenyl-1,3-propanediol dicarbamate in the compositions may be varied over wide limits and the quantity of medicament furnished by each individual tablet or capsule is relatively unimportant since the indicated total daily dose can be reached by administering either one or a plurality of capsules or tablets. However, for convenience in manufacturing and ease of administration, it is preferable that each dosage form contains at least 25 milligrams and up to 500 milligrams of 2-phenyl-1,3-propanediol dicarbamate per unit dosage form.

The present invention will now be further described and illustrated with reference to the following Examples (Examples 3 and 4 are included by way of background).

### EXAMPLE 1

2-phenyl-1,3-propanediol dicarbamate is constituted into 500 mg dosage units by encapsulation without an adjuvant into hard gelatin capsules. The yield from 1000 g of 2-phenyl-1,3-propanediol dicarbamate is about 2000 capsules each containing 500 mg of medicant.

### EXAMPLE 2

A tableting formulation is prepared as follows:
- 83 g: 2-phenyl-1,3-propanediol dicarbamate
- 13 g: powdered sugar with 3% starch
- 76 g: corn syrup
- q.s.: water
- 13 g: talc U.S.P. powdered Italian
- 3 g: magnesium stearate
- q.s.: alcohol
flavouring
The formulation is compressed into tablets, each containing 200 mg of 2-phenyl-1,3-propanediol dicarbamate. The yield is about 1750 tablets.

It has been found that the antiepileptic spectrum of Felbamate is effective in the treatment of complex partial and secondarily generalized seizures, seizure types frequently resistant to antiepileptic drug therapy. Felbamate has been found to abolish the hind limb tonic extensor phase of maximal electroshock seizures in laboratory animals and to elevate the threshold to seizures induced chemically by subcutaneous pentylenetetrazole. Felbamate has also been found to inhibit chemically-induced seizures secondary to picrotoxin. The mechanism of Felbamate's action is unknown.

Peak plasma concentration of Felbamate occurs from 1 to 3 hours after oral ingestion. In man approximately 90% of the dose of felbamate is recoverable in urine over a 10-day period. The major metabolic pathways in both animals and man are hydroxylation and conjugation.

Generally, antiepileptic drug clinical trials in patients with uncontrolled complex partial seizures and secondarily generalized seizures present difficulties since such patients may be taking one or more antiepileptic drugs which cannot usually be tapered without inducing clinically unacceptable seizure exacerbation; on the other hand, if concomitant drug administration is permitted, it may lead to pharmacologic interactions which can either vitiate the interpretation of a clinical trial, or lead to unacceptable toxicity.

In order to demonstrate the effectiveness of felbamate and, in order to overcome these obstacles as far as possible, the following procedure is followed: All patients are stabilized on carbamazepine, a present drug of choice in control of temporal lobe and grand mal epilepsy, before entry. Maintenance carbamazepine therapy is thought to give patients a reasonable degree of seizure control throughout the study, reduce the chance of drug interactions, and to allow a reasonable opportunity for felbamate to show its effect. A baseline period is used to exclude patients with evidence of seizure clustering. To ensure accurate recording of seizures, the patients remain in the hospital throughout the trial.

The study begins with a stabilization period, during which patients' therapy is adjusted to carbamazepine alone. Stabilization is considered to be achieved when the patient has two morning plasma levels of carbamazepine in the range of 4 to 16 milligrams per litre (mg/L).

Following stabilization, patients are admitted to the hospital for a three week baseline period. In order to qualify for randomization, patients must have at least six seizures during baseline, with at least one seizure in every week, and at least two weeks with two or more seizures.

Patients who meet the criterion are randomized at the end of baseline to one of four treatment sequences: felbamate-placebo-felbamate, felbamate-placebo-placebo, placebo-felbamate placebo, and placebo-felbamate-felbamate or for short FPF, FPP, PFP and PFF. The treatments are administered over the course of alternating titration and analysis periods, each lasting two weeks. During the first titration period, the patient received gradually increasing doses of felbamate or placebo. The target felbamate dose was 3000 mg/day (maximum 50/mg/kg/day). The patient was then observed on a steady dose for a two week analysis period. This was followed by two more titration-analysis period pairs.

The study design called for 28 completed patients (7 in each arm) and a one-sided test of the hypothesis of no felbamate effect. The study was planned to have a power of 0.71 to detect a 50% reduction in seizure frequency due to felbamate, at a significance level of 0.05. However, the observed variability in the data was less than the value used in planning the study, so the actual power to detect a 50% reduction was over 0.95.

Felbamate (2-phenyl-1,3-propanediol dicarbamate) has a very favourable preclinical profile characterized by a substantial margin of safety (protective index 16.9 to 19.1). The following Example 3 presents the results from a double-blind randomized clinical trial in patients with partial seizures. Criteria for patient entry into the study were 4 or more complex partial seizures per month in spite of treatment with both phenytoin and carbamazepine.

### EXAMPLE 3

Fifty-six patients (mean age 31.4 years; male=32, female=24) completed the study. The mean seizure frequencies for the eight week periods analyzed were: baseline=39.8; felbamate=34.9; placebo=40.2. Felbamate was significantly superior to placebo by percent seizure reduction (p=0.018) and truncated percent seizure reduction (p=0.007).

The mean felbamate dose was 2300 mg/day. Plasma felbamate concentrations ranged from 18.4 to 51.9, mean=32.5 mg/ml.

Adverse effects were minor and consisted of nausea and CNS effects.

In patients with complex partial seizures, the entry criteria required at least six seizures in a three week baseline period (and no more than one week with a single seizure) on carbamazepine alone. Twenty eight subjects were tested as demonstrated in Example 4.

### EXAMPLE 4

The daily dose of 50 milligrams per kilogram (maximum 3000 mg) felbamate per day was well-tolerated by all 28 completers. Only mild side effects were encountered during the trial. Felbamate reduced carbamazepine level (P 0.0001; 95% confidence interval -28%, -20%). There was no significant difference in seizure frequency between placebo and felbamate periods (one-sided P=O.172).

The Lennox-Gastaut syndrome is characterized by mental retardation, slow spike-and-wave EEG and intractable seizures. Example 5, which follows, demonstrates the effectiveness of Felbamate in the treatment of Lennox-Gastaut syndrome.

### EXAMPLE 5

Six patients ranging in age from 6 to 30 years (average 14.5 years) were treated with Felbamate. All of the patients had completed a double-blind study, were on two standard antiepileptics and were having a minimum of 90 seizures/month. Treatment length ranged from 1 to 4 months. Felbamate dose ranged between 45 and 58 mg/kg/day (maximum dose 3600 mg/day). Two of the subjects experienced a 50% reduction of seizures and three of the subjects had a 75 to 100% reduction of seizures. Two patients experienced at least one or more seizure-free weeks.

In conclusion, results suggest that Felbamate is an effective treatment for the seizures associated with the Lennox-Gastaut syndrome.

Indeed, the superiority of felbamate over placebo in a population of persons with severely refractory epilepsy indicates this medication to be a major anti-epileptic agent.

## Claims

1. The use of 2-phenyl-1,3-propanediol dicarbamate for the manufacture of a medicament for the prevention and control of seizures associated with Lennox-Gastaut syndrome.

2. A use according to claim 1, wherein the medicament is manufactured in the form of a dosage unit comprising from 25 to 500 milligrams 2-phenyl-1,3-propanediol dicarbamate per dosage unit and a pharmaceutical carrier.

3. A use according to claim 1, wherein the medicament is manufactured in the form of a gelatin capsule containing from 25 to 500 milligrams 2-phenyl-1,3-propanediol dicarbamate.

4. A use according to claim 1, wherein the medicament is manufactured in the form of a tabletted composition comprising a solid pharmaceutical carrier and from 25 to 500 milligrams 2-phenyl-1,3-propanediol dicarbamate.

5. A use according to claim 1, wherein the medicament is to be administered to a warm-blooded animal in a daily dosage of from 100 milligrams to 5 grams 2-phenyl-1,3-propanediol-dicarbamate.

## Patentansprüche

1. Verwendung von 2-Phenyl-1,3-propandiol-dicarbamat zur Herstellung eines Medikamentes für die Verhütung und Kontrolle von Anfällen, die mit dem Lennox-Syndrom einhergehen.

2. Verwendung nach Anspruch 1, worin das Medikament in der Form einer Dosierungseinheit hergestellt wird, das von 25 mg bis 500 mg 2-Phenyl-1,3-propandiol-dicarbamat pro Dosierungseinheit und einen pharmazeutischen Träger umfaßt.

3. Verwendung nach Anspruch 1, worin das Medikament in der Form einer Gelatinekapsel hergestellt wird, die von 25 mg bis 500 mg 2-Phenyl-1,3-propandiol-dicarbamat enthält.

4. Verwendung nach Anspruch 1, worin das Medikament in der Form einer tablettierten Zusammensetzung hergestellt wird, die einen festen pharmazeutischen Träger und von 25 mg bis 500 mg 2-Phenyl-1,3-propandiol-dicarbamat umfaßt.

5. Verwendung nach Anspruch 1, worin das Medikament einem warmblütigen Tier in einer täglichen Dosierung von 100 mg bis 5 g 2-Phenyl-1,3-propandiol-dicarbamat verabreicht werden soll.

## Revendications

1. L'utilisation de 2-phényle-1, 3-propanediol dicarbamate pour fabriquer un médicament de prévention et de contrôle des crises associées au syndrome de Lennox-Gastaut.

2. Une utilisation selon la revendication 1, dans laquelle le médicament est fabriqué sous forme d'une dose posologique comprenant de 25 à 500 milligrammes de 2-phényle-1, 3-propanediol dicarbamate par unité posologique et un porteur pharmaceutique.

3. Une utilisation selon la revendication 1, dans laquelle le médicament est fabriqué sous forme d'une capsule de gélatine contenant de 25 à 500 milligrammes de 2-phényle-1, 3-propanediol dicarbamate.

4. Une utilisation selon la revendication 1, dans laquelle le médicament est fabriqué sous forme d'une composition en comprimés comprenant un porteur pharmaceutique solide et de 25 à 500 milligrammes de 2-phényle-1, 3-propanediol dicarbamate.

5. Une utilisation selon la revendication 1, dans laquelle le médicament doit être administré à un animal à sang chaud en une dose quotidienne de 100 milligrammes à 5 grammes de 2-phényle-1, 3-propanediol dicarbamate.
